(19) Europäisches Patentamt
European Patent Office
Office européen des brevets

(11) Publication number: 0 221 588 A2

# (12) EUROPEAN PATENT APPLICATION

(21) Application number: 86201711.8

(22) Date of filing: 12.05.83

(51) Int. Cl.[4]: C 07 D 489/02
C 07 J 43/00, C 07 D 401/12
C 07 D 213/80, C 07 D 211/9-
0
C 07 H 19/04, A 61 K 47/00

(30) Priority: 18.05.82 US 379316
27.01.83 US 461543
15.03.83 US 475493

(43) Date of publication of application:
13.05.87 Bulletin 87/20

(84) Designated Contracting States:
AT BE CH DE FR GB LI LU NL SE

(60) Publication number of the earlier application in accordance with Art. 76 EPC: 0 110 955

(71) Applicant: UNIVERSITY OF FLORIDA
207 Tigert Hall
Gainesville Florida 32611(US)

(72) Inventor: Bodor, Nicholas S.
7211 South West 97th Lane
Gainesville Florida 32608(US)

(74) Representative: Pendlebury, Anthony et al,
Page, White & Farrer 5 Plough Place New Fetter Lane
London EC4A 1HY(GB)

## (54) Brain-specific drug delivery.

(57) The invention provides compounds adapted for the site-specific/sustained delivery of a centrally acting drug species to the brain having the formula [D-DHC] and the non-toxic pharmaceutically acceptable salts thereof, wherein [D] is a centrally acting drug species which is an antiviral agent, an antiinflammatory steroid, a narcotic or an estrogen and which contains at least two reactive hydroxyl functional groups, and [DHC] is the reduced, biooxidizable, blood-brain barrier penetrating lipoidal form of a dihydropyridine⇌pyridinium salt redox carrier comprising at least one dihydropyridine ring system of the formula

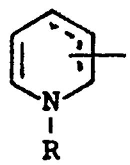

wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species. The corresponding quaternary derivatives are also described.

EP 0 221 588 A2

# BRAIN-SPECIFIC DRUG DELIVERY

## Field of the Invention:

The present invention relates to a dihydropyridine/pyridinium salt type of redox system for the site-specific or sustained delivery (or both) of a wide variety of drug species to the brain. More especially, this invention relates to the discovery that a biologically active compound coupled to a lipoidal carrier moiety comprising a dihydropyridine nucleus readily and easily penetrates the blood-brain barrier ("BBB") and attains increased levels of concentration in the brain; oxidation of the dihydropyridine carrier moiety *in vivo* to the ionic pyridinium salts prevents its elimination from the brain, while elimination from the general circulation is accelerated, resulting in significant and prolongedly sustained brain-specific drug activity, whether ascribable to the cleavage of the $[D\text{-}QC]^+$ entity and sustained release of the drug in the brain and/or to $[D\text{-}QC]^+$ itself.

## Background Art:

The delivery of drug species to the brain is ofttimes seriously limited by transport and metabolism factors and, more specifically, by the functional barrier of the endothelial brain capillary wall deemed the blood-brain barrier, BBB. Site-specific delivery and sustained delivery of drugs to the brain are even more difficult, and to date no useful simple or generic techniques to achieve such phenomena are known to the art.

Indeed, the barriers separating plasma from the brain and cerebrospinal fluid (CSF) are complex systems involving passive and active transport and subserve a number of important functions. The boundary between plasma and the central nervous system (CNS) is much less permeable than that between plasma and other tissue cells to a variety of water soluble substances, such as organic electrolytes, organic acids and bases, as well as to large molecules such as proteins. Such a barrier also provides a path for clearance from the brain of the breakdown products of cellular metabolism. The CNS and its fluids can be considered basically a three-compartment system: the blood or the plasma, CSF and brain tissue. There is a diffusion-controlled exchange between CSF and the extracellular fluid (CF) of the brain. It has also been suggested that the permeabilities of blood-CSF and blood-brain barriers are practically identical with respect to drugs and other foreign substances. Mayer et al, J. Pharmacol. and Exp. Therap., 125, 185 (1959).

The BBB is, moreover, basically the result of the fact that the endothelial cells in the brain capillaries are joined by continuous, tight intercellular junctions, such that material has to pass through the cells rather than between them in order to move from blood to brain. It is interesting that there are areas within the brain, such as the subfornical body and the postremia in which the capillary cells are not closely linked so that they lack the characteristics of the BBB. They provide the entry of small amounts of compounds which would not ordinarily enter the barriers. Hoffmann and Olszewzki, Neurology (Minneap.), 11, 1081 (1961).

Foreign compounds which enter organs other than the central nervous system with ease, may penetrate the CNS slowly or hardly at all. A number of theories concerning the nature of the barrier have been proposed. The widely accepted concept describes the boundary as a fat-like layer interspersed with small pores, although the BBB is not a simple, anatomically well-defined unitary physical entity. Shuttleworth, Prog. Exp. Tumor Res., 17, 279 (1972). Penetration of such a barrier may occur by several processes: lipid soluble substances may passively penetrate into the cells, while small molecules such as water and urea may pass through the pores. In addition to these simple physical processes, carrier-mediated and active transport processes govern the movement of many molecules through the BBB. Thus, it is generally accepted that lipid solubility, degree of ionic dissociation or protonation and the ability of temporary combination with membrane constituents affect delivery through the BBB. It has been shown, for example, that in the class of barbiturates, a quantitative correlation could be established between their ease to pass into the brain (as reflected by the different times of onset of anesthetic action) and their lipid/water partition coefficient. Mark et al, J. Pharmacol. and Exp. Therap., 123, 79 (1957). The role of lipid solubility in drug penetration through the BBB is also exemplified by the better absorption of the sparingly water-soluble thiamine propyl disulfide (TPD) as compared to the water-soluble thiamine hydrochloride (THCl). Thomson et al, Ann. Int. Med., 74, 529 (1971). Some materials such

as glucose and amino acids are transported by active mechanism, characterized by saturation, bidirectional molecular specificity, bidirectional competitive inhibition and bidirectional countertransport. Fishman, Am J. Physiol., 206, 836 (1964).

Changes in permeability of the BBB can be caused by several pathological and toxicological processes. Pardridge, Connor and Crawford, CRC Crit. Rev. Toxicol., 179 (1975). A general increase in the barrier permeability, such as a nonspecific breakdown of the barrier has, however, severe consequences, including cerebral edema.

It too is well documented that the BBB is relatively impermeable to the ionized forms of drugs and other molecules. Drugs which are weak organic electrolytes appear to pass from blood to CSF to reach a steady state ratio characteristic of each molecule according to its $pK_a$ and the existence of a normal pH gradient between blood and CSF. It is clear that it is the most difficult for quaternary pyridinium or ammonium salts to penetrate the BBB.

And removal of substances from the brain and CSF is obviously a significant factor in regulating drug concentrations in the CNS. There are several efflux processes: bulk flow via the arachnoid villi, diffusion of lipid soluble substances into brain and blood, active transport and metabolism by adjacent meninges. Once a drug or metabolite enters the CSF from blood or brain by simple diffusion, it may rapidly be removed, either by nonselective bulk flow or by active transport mechanism associated with the choroid plexus or other nondefined structures in the CSF compartment. It is generally accepted that highly lipid-soluble drugs

leave the CSF more rapidly than poorly lipid-soluble ones, but the barrier to passage of compounds from CSF has only superficial similarity to the blood-CSF barrier.

Drug elimination processes from the brain are significantly directly related to drug accumulation in the brain. It is generally assumed that efflux in the opposite direction involves almost the same processes as for entry, except that the role of the bulk flow and the metabolic processes in the brain are not to be overlooked.

The two elimination processes studied in the earlier literature and which can be said to have a certain bearing on the present invention involve elimination from the brain of ionic species. Thus, it is found that non-metabolized ionic species, such as the acetate ion, have a three times slower elimination rate from the CSF than from the blood. Freundt, Arz., Forsch., 23, 949 (1973). An even more dramatic change in the elimination rate was found in the case of a quaternary piperidinium salt. The quaternary salt, formed *in situ* after delivery of a haloalkylamine, which undergoes cyclization to the quaternary salt, in the brain, as well, was found to have an at least ten times slower elimination rate from the brain than from the rest of the body. It was concluded by the authors (Ross and Froden, Eur. J. Pharmacol., 13, 46 [1970] that the outflow rate of the quaternary salt corresponded to the inflow rate. Similar results were obtained for the erythrocytes: the efflux of the quaternary salt was very slow. Ross, J. Pharm. Pharmacol., 27, 322 (1975).

And while it too has been suggested to deliver a drug species, specifically N-methylpyridinium-2-carbaldoxime chloride (2-PAM), into the brain, the active nucleus of which in and of itself constituting a quaternary pyridinium salt, by way of the dihydropyridine latentiated prodrug form thereof, such approach is conspicuously delimited to relatively small molecule quaternary pyridinium ring-containing drug species and does not provide the overall ideal result of brain-specific, sustained release of the desired drug, with concomitant rapid elimination from the general circulation, enhanced drug efficacy and decreased toxicity. Hence, no "trapping" in the brain of the 2-PAM formed _in situ_ results, and obviously no brain-specific, sustained delivery occurs as any consequence thereof: the 2-PAM is eliminated as fast from the brain as it is from the general circulation and other organs. Compare U.S. Patents Nos. 3,929,813 and 3,962,447; Bodor et al, _J. Pharm. Sci._, _67_, No. 5, 685 (1978). It has also been speculated to deliver, e.g., an antitumor agent into the brain by utilizing a dihydropyridine/pyridinium redox carrier moiety therefor, but this particular hypothesis necessarily entails derivatizing the dihydropyridine/pyridinium carrier with a substituent $R_1$ itself critically designed to control the release rate of the active drug species from the quaternary drivative thereof, as well as being critically functionally coordinated with the particular chemical and therapeutic activity/nature of the antitumor drug species itself; Bodor et al, _J. Pharm. Sci._, supra.

Accordingly, acutely serious need exists in this art for a truly effective generic but nonetheless flexible method for the site-specific, or sustained delivery, or both, of drug species to the brain, while at the same time avoiding the aforesaid noted and notable disadvantages and drawbacks associated with penetration of the blood-brain barrier, with dihydropyridine latentiated prodrug forms of drug species themselves comprising a pyridinium salt active nucleus, and with the necessity for introducing critically coordinated and designed, release rate-controlling substituents onto any particular drug carrier moiety.

Recently, Bodor et al, Science, Vol. 214, December 18, 1981, pp. 1370-1372, have reported on site-specific sustained release of drugs to the brain. The Science publication outlines a scheme for specific and sustained delivery of drug species to the brain, as depicted in the following Scheme 1:

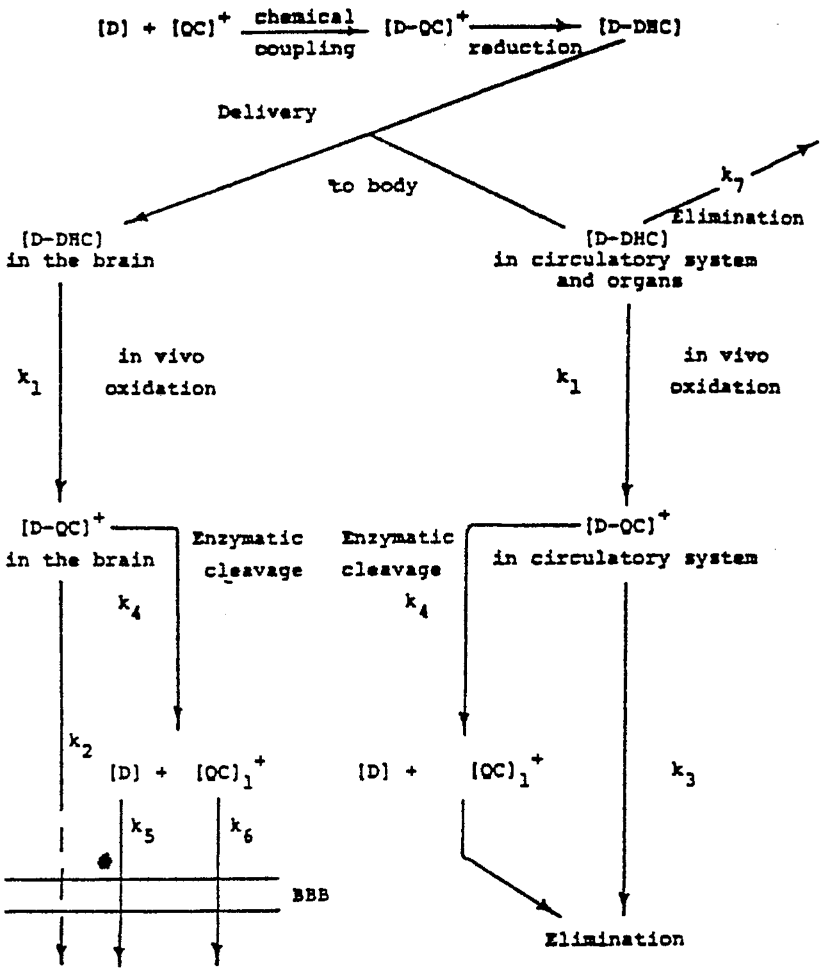


Scheme 1: BBB, blood-brain barrier.

According to the scheme in Science, a drug [D] is coupled to a quaternary carrier $[QC]^+$ and the $[D-QC]^+$ which results is then reduced chemically to the lipoidal dihydro form [D-DHC]. After administration of [D-DHC] in vivo, it is rapidly distributed throughout the body, including the brain. The dihydro form [D-DHC] is then in situ oxidized (rate constant, $k_1$) (by the NAD $\rightleftharpoons$ NADH system) to the ideally inactive original $[D-QC]^+$ quaternary salt which, because of its ionic, hydrophilic character, should be rapidly eliminated from the general circulation of the body, while the blood-brain barrier should prevent its elimination from the brain ($k_3 >> k_2$; $k_3 >> k_7$). Enzymatic cleavage of the $[D-QC]^+$ that is "locked" in the brain effects a sustained delivery of the drug species [D], followed by its normal elimination ($k_5$), metabolism. A properly selected carrier $[QC]^+$ will also be rapidly eliminated from the brain ($k_6 >> k_2$). Because of the facile elimination of $[D-QC]^+$ from the general circulation, only minor amounts of drug are released in the body ($k_3 >> k_4$); [D] will be released primarily in the brain ($k_4 > k_2$). The overall result ideally will be a brain-specific sustained release of the target drug species.

Bodor et al have reported, in <u>Science</u>, their work with phenylethylamine as the drug model, which was coupled to nicotinic acid, then quaternized to give compounds of the formula

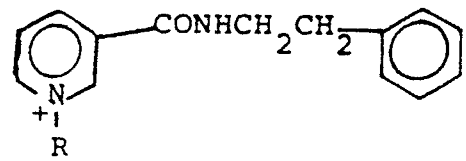 (R=$CH_3$ or $CH_2$ 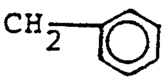 )

which were subsequently reduced by sodium dithionite to the corresponding compounds of the formula

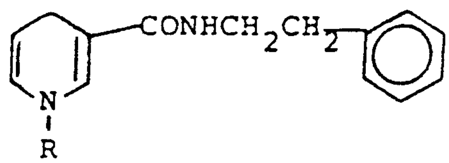 (R=$CH_3$ or $CH_2$ 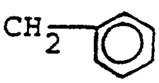 ).

Testing of the N-methyl derivative *in vivo* supported the criteria set forth in Scheme 1. Bodor et al speculated that various types of drugs might possibly be delivered using the depicted or analogous carrier systems and indicated that use of N-methylnicotinic acid esters and amides and their pyridine ring-substituted derivatives was being studied for delivery of amino- or hydroxyl-containing drugs, including small peptides, to the brain. No other possible specific carriers were disclosed.

Other reports of Bodor et al's work have appeared in <u>The Friday Evening Post</u>, August 14, 1981, Health Center Communications, University of Florida, Gainesville, Florida; <u>Chemical & Engineering News</u>, December 21, 1981, pp. 24-25; and <u>Science News</u>, January 2, 1982, Vol. 121, No. 1, page 7. These publications do not suggest any carrier systems other

than the specific N-methyl and N-benzyl nicotinic acid-type carriers disclosed in the Science publication. Other classes of drugs as well as a few specific drugs are mentioned as possible candidates for derivativization; for example, steroid hormones, cancer drugs and memory enhancers are indicated as targets for possible future work, as are enkephalins, and specifically, dopamine and testosterone. The publications do not suggest how to link such drugs to the carrier, except possibly when the drugs are simple structures containing a single $NH_2$ or, perhaps, simple structures containing a single OH, of the primary or secondary type, as is the case with phenylethylamine or testosterone. There is, for example, no suggestion of how one of ordinary skill in the art would form a drug-carrier combination when the drug has a more complicated chemical structure than phenylethylamine, e.g., dopamine or an enkephalin. Thus, except in a very limited area, where only a combination of certain types of drug structures with certain utility classes and certain carrier structures is taught or suggested, the publications simply do not make the broad concept of the invention as represented by Scheme 1 available to the public. Likewise, the many embodiments of the invention as described hereinafter are unsuggested by the art.

Summary and Objects of the Invention:

Accordingly, a major object of the present invention is the provision of a generic method for the specific and/or target enhanced delivery to the brain of a wide variety of drug species and to achieve brain-specific drug delivery by effecting the bidirectional transport of the drug species into and out of the brain employing dihydropyridine $\rightleftharpoons$ pyridinium salt carrier type redox systems.

Another object of the invention is to provide for brain specific drug delivery utilizing a dihydropyridine $\rightleftharpoons$ pyridinium salt carrier type redox system, which drug/carrier system is characterised by enhanced drug efficacy and decreased toxicity. Indeed, consistent herewith systemic toxicity is significantly reduced by accelerating the elimination of the drug/quaternary carrier system, and even central toxicity is reduced by providing a low level, sustained release of the active drug species in the brain.

Yet another object of this invention is the provision of a chemical delivery system for the site-specific and sustained release of drug species to the brain, and one in which a special pro-prodrug reduced form of an active drug species is actually delivered to the body of a patient, not a prodrug as such and not a drug-carrier entity necessarily comprised of critically tailored release rate-controlling substituent(s).

Yet another object of this invention is to provide enhanced delivery to the brain of a wide variety of centrally acting agents which are not themselves able to penetrate the blood-brain barrier to any considerable extent.

In accord with the foregoing, the present invention provides compounds adapted for the site-specific/sustained delivery of a centrally acting drug species to the brain, said compounds being:

0221588

compounds of the formula

[D-DHC] (I)

and the non-toxic pharmaceutically acceptable salts thereof, wherein [D] is a centrally acting drug species

which is an antiviral agent, an antiinflammatory steroid, a narcotic or an estrogen and which contains at least two reactive hydroxyl functional groups, and [DHC] is the reduced, biooxidizable, blood-brain barrier penetrating lipoidal form of a dihydropyridine⇄pyridinium salt redox carrier comprising at least one dihydropyridine ring system of the formula

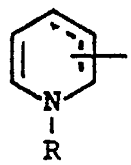

wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species.

In another aspect, the present invention provides compounds having the formula

$$[D\text{-}QC]^{+} \qquad (II)$$

wherein [D] is a centrally acting drug species which

is an antiviral agent, an antiinflammatory steroid, a narcotic or an estrogen and which contains at least two reactive hydroxyl functional groups, and $[QC]^+$ is the hydrophilic, ionic pyridinium salt form of a dihydropyridine ⇌ pyridinium salt redox carrier comprising at least one pyridinium ring system of the formula

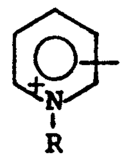

wherein R is lower alkyl or benzyl, a ring carbom atom of each pyridinium ring system being connected via a bridging group to a reactive hydroxyl functional group in the centrally acting drug species.

Detailed Description of the Invention:

More particularly in accord with the present invention, the following definitions are applicable:

The term "lipoidal" as used herein is intended to designate a carrier moiety which is lipid-soluble or lipophilic.

The expression "hydroxyl protective group" is intended to designate a group which prevents premature metabolism of an OH group or groups prior to the compound's reaching the desired site in the body. Typical hydroxyl protective groups contemplated by the present invention are acyl groups and carbonates.

When the hydroxyl protective group is acyl (i.e., when it is an organic radical derived from a carboxylic acid by removal of the hyroxyl group), it preferably represents an acyl radical selected from the group consisting of alkanoyl having 2 to 8 carbon atoms; alkenoyl having one or two double bonds and 3 to 8 carbon atoms;

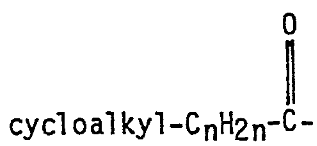

wherein the cycloalkyl portion contains 3 to 7 ring atoms and n is zero, one, two or three; phenoxyacetyl; pyridinecarbonyl; and

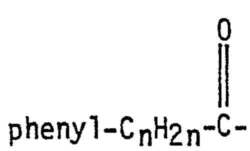

wherein n is zero, one, two or three and phenyl is unsubstituted or is substituted by 1 to 3 alkyl ________________________________

each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms.

When the acyl group is alkanoyl, there are included both unbranched and branched alkanoyl, for example, acetyl, propionyl, butyryl, isobutyryl, valeryl, isovaleryl, 2-methylbutanoyl, pivalyl (pivaloyl), 3-methylpentanoyl, 3,3-dimethylbutanoyl, 2,2-dimethylpentanoyl and the like. Pivalyl and isobutyryl are especially preferred.

When the acyl group is alkenoyl, there are included, for example, crotonyl, 2,5-hexadienoyl and 3,6-octadienoyl.

When the acyl group is

$$\text{cycloalkyl-}C_nH_{2n}\text{-}\overset{\overset{\displaystyle O}{\|}}{C}\text{-}$$

there are included cycloalkanecarbonyl and cycloalkanealkanoyl groups wherein the cycloalkane portion can optionally bear 1 or 2 alkyl groups as substituents, e.g. cyclopropanecarbonyl, 1-methylcyclopropanecarbonyl, cyclopropaneacetyl, α-methylcyclopropaneacetyl, 1-methylcyclopropaneacetyl, cyclopropanepropionyl, α-methylcyclopropanepropionyl, 2-isobutylcyclopropanepropionyl, cyclobutanecarbonyl, 3,3-dimethylcyclobutanecarbonyl, cyclobutaneacetyl, 2,2-dimethyl-3-ethylcyclobutaneacetyl, cyclopentanecarbonyl, cyclohexaneacetyl, cycloheptanecarbonyl and cycloheptanepropionyl.

When the acyl group is pyridinecarbonyl, there are included picolinoyl (2-pyridinecarbonyl), nicotinoyl (3-pyridinecarbonyl) and isonicotinoyl (4-pyridinecarbonyl).

When the acyl group is

$$\text{phenyl-}C_nH_{2n}\text{-}\overset{\overset{\displaystyle O}{\|}}{C}\text{-}$$

there are included, for example, benzoyl, phenylacetyl, α-phenylpropionyl, β-phenylpropionyl, p-toluyl, m-toluyl, o-toluyl, o-ethylbenzoyl, p-tert-butylbenzoyl, 3,4-dimethylbenzoyl, 2-methyl-4-ethylbenzoyl, 2,4,6-trimethylbenzoyl, m-methylphenylacetyl, p-isobutylphenylacetyl, β-(p-ethylphenyl)-propionyl, p-anisoyl, m-anisoyl, o-anisoyl, m-isopropoxybenzoyl, p-methoxyphenylacetyl, m-isobutoxyphenylacetyl, m-diethylaminobenzoyl, 3-methoxy-4-ethoxybenzoyl, 3,4,5-trimethoxybenzoyl, p-dibutylaminobenzoyl, p-n-butoxybenzoyl, 2,4,6-triethoxybenzoyl, 3,4-diethoxyphenylacetyl, β-(3,4,5-trimethoxyphenyl)propionyl, o-iodobenzoyl, m-bromobenzoyl, p-chlorobenzoyl, p-fluorobenzoyl, 2-bromo-4-chlorobenzoyl, 2,4,6-trichlorobenzoyl, p-chlorophenylacetyl, α-(m-bromophenyl)propionyl, p-trifluoromethylbenzoyl, 2,4-di(trifluoromethyl)benzoyl, m-trifluoromethylphenylacetyl, β-(p-trifluoromethylphenyl)propionyl, 2-methyl-4-methoxybenzoyl, 3-chloro-4-ethoxybenzoyl, β-(3-methyl-4-chlorophenyl)propionyl, p-dimethylaminobenzoyl, p-(N-methyl-N-ethylamino)benzoyl, o-acetamidobenzoyl, m-propionamidobenzoyl, 3-chloro-4-acetamidophenylacetyl and p-acetamidophenylpropionyl.

When the hydroxyl protective group is a carbonate grouping, it has the structural formula

$$Y'-O-\overset{\overset{\large O}{\|}}{C}-$$

i.e., it is an organic radical which can be considered to be derived from a carbonic acid by removal of the hydroxyl group from the COOH portion. Y' preferably represents alkyl having 1 to 7 carbon atoms; alkenyl having one or two double bonds and 2 to 7 carbon atoms;

$$\text{cycloalkyl-}C_nH_{2n}-$$

wherein the cycloalkyl portion contains 3 to 7 ring atoms and n is zero, one, two or three; phenoxy; 2-, 3- or 4-pyridyl; or

$$\text{phenyl-}C_nH_{2n}-$$

wherein n is zero, one, two or three and phenyl is unsubstituted or is substituted by 1 to 3 alkyl each having 1 to 4 carbon atoms, alkoxy having 1 to 4 carbon atoms, halo, trifluoromethyl, dialkylamino having 2 to 8 carbon atoms or alkanoylamino having 2 to 6 carbon atoms. Most preferably, Y' is $C_1$-$C_7$ alkyl, particularly ethyl or isopropyl.

Similarly, the expression "carboxyl protective group" is intended to designate a group which prevents premature metabolism of a COOH group or groups prior to the compound's reaching the desired site in the body. Typical carboxyl protecting groups are the

groups encompassed by Y' above, especially $C_1$-$C_7$ alkyl, particularly ethyl or isopropyl.

By "centrally acting" drug species, active agent or compound as utilized herein, there is of course intended any drug species or the like, the principal pharmacological activity of which is CNS and a result of direct action in the brain.

Exemplary such centrally acting drug species are strong analgesics and narcotics, narcotic antagonists, and the steroid hormone estradiol.

Illustrative ultimate species of centrally active drug entities are: oxycodone, morphine and oxymorphone, which are narcotic analgesics; nalorphine and naloxone, narcotic antagonists; estradiol, and estriol, the natural estrogens; apomorphine, a narcotic depressant; ethinyl estradiol, an estrogen; ribavirin, an antiviral agent; and benzestrol and diethylstilbestrol, synthetic estrogens.

Preferred classes of centrally acting drugs for use herein are the steroids, and antiviral agents. Among the steroids, there can be mentioned anti-inflammatory adrenal cortical steroids such as hydrocortisone, betamethasone, cortisone, dexamethasone, flumethasone, fluprednisolone, meprednisone, methyl prednisolone, prednisolone, prednisone, triamcinolone, cortodoxone, fludrocortisone, flurandrenolone acetonide paramethasone and the like; and estrogens e.g., ethinyl estradiol, estradiol, estriol, and the like. Among the antiviral agents, there can be mentioned ribavirin; bridgehead C-nucleosides such as 5,7-dimethyl 2-ß-D-ribofuranosyl-s-triazole (1,5-a)pyrimidine; glycosides such as 2-deoxy-D-glucose, glucosamine, 2-deoxy-2-fluoro-D-mannose and 6-amino-6-deoxy-D-glucose; phenyl glucoside derivatives such as phenyl-6-chloro-6-deoxy-ß-D-glucopyranoside; (S)-9-(2,3-dihydroxypropyl)adenine; 6-azauridine; and 5,6-dichloro-1-ß-D-ribofuranosylbenzimidazole.

It too will be appreciated that by "dihydropyridine carrier" or "[DHC]", there is intended any nontoxic carrier moiety as hereinbefore defined, comprising, containing or including the dihydropyridine nucleus, the only criterion therfor being capacity for BBB penetration and *in vivo* oxidation thereof to the corresponding quaternary pyridinium salt carrier $[QC]^+$. As aforesaid, the ionic pyridinium salt drug/carrier prodrug entity $[D\text{-}QC]^+$ which results from such *in vivo* oxidation is prevented from efflux from the brain, while elimination from the general circulation is accelerated. Subsequently, the covalent or equivalent bond coupling the drug species [D] to the quaternary carrier $[QC]^+$ is metabolically cleaved which results in sustained delivery of the drug [D] in the brain and facile elimination of the carrier moiety $[QC]^+$. Such "covalent or equivalent bond" between the drug and the quaternary carrier can be a simple direct chemical bond, e.g., an amide, an ester, or any other like bona, or same can even be comprised of a linking group or function, e.g., a thiazolidine bridge or a peptide linkage. Nonetheless, the bond in the formulae $[D\text{-}QC]^+$ and [D-DHC] is intended to be and is hereby defined as inclusive of all such alternatives. And the cleavage of the $[D\text{-}QC]^+$ prodrug to sustaineedly deliver the drug species [D] in the brain with concomitant facile elimination of the carrier moiety $[QC]^+$ is characteristically enzymatic cleavage, e.g., by esterase, amidase, cholinesterase, hydrolytic enzyme, or peptidase, albeit any type of *in brain* cleavage which might result, whether enzymatic, metabolic or otherwise, of course remains within the ambit of this invention. Thus, the drug release rate controlling parameter of the subject pro-prodrugs is imparted simply via the cleavable bonding between drug and carrier, and not by any release rate controlling substituent(s).

The expression "non-toxic pharmaceutically acceptable salts" as used herein generally includes the nontoxic salts of compounds of formula (I), wherein [D] is a centrally acting drug species and [DHC] is the reduced, biooxidizable, blood-brain barrier penetrating form of a dihydropyridine $\rightleftharpoons$ pyridinium salt redox carrier as

hereinbefore defined formed with nontoxic, pharmaceutically acceptable inorganic or organic acids HX. For example, the salts include those derived from inorganic acids such as hydrochloric, hydrobromic, sulfuric, sulfamic, phosphoric, nitric and the like; and the salts prepared from organic acids such as acetic, propionic, succinic, glycollic, stearic, lactic, malic, tartaric, citric, ascorbic, pamoic, maleic, hydroxymaleic, phenylacetic, glutamic, benzoic, salicylic, sulfanilic, fumaric, methanesulfonic, toluene-sulfonic and the like.

In one embodiment according to this invention, simple nontoxic carrier systems $[D\text{-}QC]^+ \rightleftharpoons [D\text{-}DHC]$ are envisaged, utilizing a wide variety of models for D, such as those above outlined. Representative such carrier systems and models are:

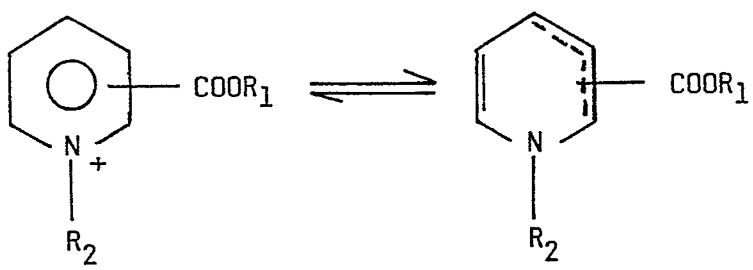

$R_1O-$ = D

wherein $R_2$ is simply alkyl, e.g., $CH_3$, or benzyl, albeit vitually any other effective substituent is intended. Exemplary of such simple carrier systems are N-alkyl nicotinate ester derivatives, tethered to the drug species. The trigonelline (N-methylnicotinic acid) system is quite effective as a carrier; it also is readily eliminated from the circulation and is virtually non-toxic.

The present invention provides a flexible arsenal of dihydropyridine $\rightleftharpoons$ pyridinium salt redox carriers are defined herein for the site-specific/sustained delivery of virtually any

centrally acting drug species to the brain. Moreover, any dihydropyridine/pyridinium salt redox carrier entity is contemplated and inteded hereby generically, and any such carrier moiety need not be, and is not derivatized with a drug release rate controlling substituent critically tailored to meet, or be coordinated with, the chemical nature and delivery requirements of the particular drug species sought to be preferentially administered to the brain or testes. As utilized herein, the term "carrier" is to be understood as connoting just such a non-derivatized, non-drug/carrier coordinated entity, for consistent herewith it is the "carrier" entity itself and not the nature of any activity or release rate controlling/modifying substituent which is responsible for providing the desired brain-specific or testicular-specific result.

Generally preferred dihydropyridine $\rightleftharpoons$ pyridinium salt redox carriers for use in the present invention include the following pyridinium systems (where D represents the drug), and obviously the corresponding dihydro forms:

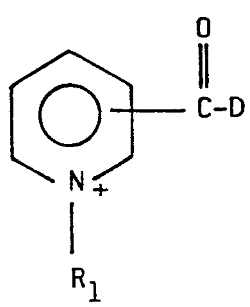

(i)

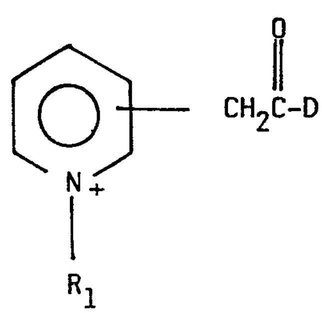

(ii)

and

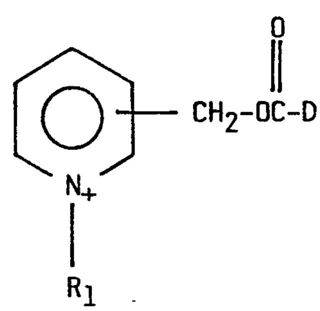

(iii)

in which the depicted substituent is in the 2-, 3- or 4- position, and $R_1$ is $C_1$-$C_7$ alkyl or $C_7$-$C_{10}$ aralkyl, preferably methyl or benzyl.

The corresponding dihydro forms of the foregoing preferred pyridinium salts are depicted below, wherein the position and identity of the structural variables are as indicated above:

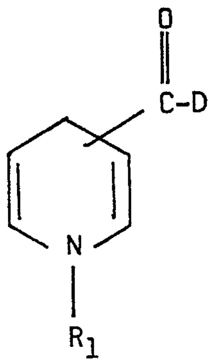

(i)

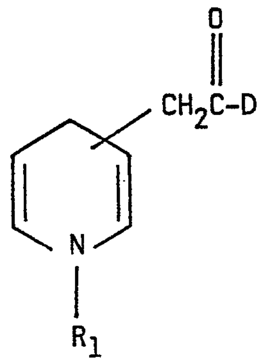

(ii)

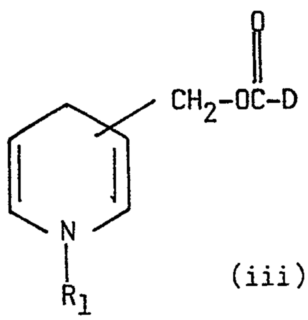

(iii)

In one embodiment of the delivery system, applicable for any drug species to be linked to such a carrier, either directly or indirectly, i.e., mediated by a carboxylic acid, e.g., succinic acid, or other linkage, provided is a mono- or poly-substituted nontoxic polyol (such as inositol or sugars) having the trigonelline $\rightleftharpoons$ dihydrotrigonelline system and the compounds to be delivered linked to the same molecule.

Various illustrative synthetic schemes as applied to specific centrally acting drugs in accord with this invention are set forth below in the section entitled "Illustrative Synthetic Methods". While the sequence of reaction steps can be varied in many cases, in general, the final step (except in the case of optional salt formation) will be reduction of a pyridinium compound of formula (II) to the corresponding dihydropyridine compound of formula (I). The

reduction is usually conducted at a temperature from about -10°C to room temperature, for a period of time from about 10 mins to 2 hrs, conveniently at atmospheric pressure. Typically, a large excess of reducing agent is employed, e.g., a 1:5 molar ratio of reducing agent to starting [D-QC]$^+$ compound. The process is conducted in the presence of a suitable reducing agent, preferably an alkali metal dithionite such as sodium dithionite or an alkali metal borohydride such as sodium borohydride or lithium aluminum borohydride, in a suitable solvent. Sodium dithionite reduction is conveniently carried out in an aqueous solution; the dihydro product [D-DHC] is usually insoluble in water and thus can be readily separated from the reaction medium. In the case of sodium borohydride reduction, an organic reaction medium is employed, e.g., a lower alkanol such as methanol, an aqueous alkanol or other protic solvent.

Suitable nontoxic pharmaceutically acceptable carriers (i.e. diluentes or vehicles) for use with the topic compounds [D-DHC], e.g., those less toxic than the target drug species themselves, will be apparent to those skilled in this art. Compare, for example, Remington's Pharmaceutical Sciences, 4th Edition (1970). Obviously, the choice of suitable carriers (i.e. diluentes or vehicles) will depend upon the exact nature of the particular dosage form selected, as well as upon the identity of the active drug species [D]. The therapeutic dosage ranges for administration of the compounds according to this invention will generally be the same as, or less than, those characteristically used in this art for administration of the known drug species [D], per se. Naturally, such therapeutic dosage ranges will vary with the size of the patient, the condition for which the [D-DHC] compound is administered, the particular dosage form employed, and the like. The quantity of given dosage form needed to deliver the desired dose of [D] will of course depend upon the concentration of [D-DHC] in any given pharmaceutical composition/dosage form thereof.

The ability of the topic compounds to cross the BBB and to be "locked into" the brain allows administration of the drug in a site-specific manner. A combination of the present dihydropyridine $\rightleftharpoons$ pyridinium salt redox system with a sustained release system will further enhance this site-specificity. Thus, a preferred embodiment of the invention comprises formulating the [D-DHC] compound or the salt of a [D-DHC] compound utilizing a sustained release carrier (i.e. diluent or vehicle) system and/or route of administration capable of slowly releasing the chemical, e.g., sustained release tablets and capsules for oral administration; subcutaneous injection, or implantation of drugs in solid pellet form (for example, distributed in a biodegradable polymer); intramuscular injection of the compound in solution in oil or suspended in a repository vehicle; a transdermal delivery device or form such as an ointment to be applied locally to the desired site (when the drug is susceptible of delivery through the skin) and the like. The rate of release of compound from the sustained release system should be comparable to the rate of *in vivo* oxidation of the dihydro form of the redox system in order to achieve the greatest degree of enhancement of specificity.

## Method A

The drug is reacted with nicotinoyl chloride, with nicotinic anhydride, or with nicotinic acid in the presence of a suitable dehydrating agent such as dicyclohexylcarbodiimide, in an appropriate organic solvent, to afford the corresponding nicotinate. The nicotinate is then quaternized, typically by treatment with methyl iodide in a suitable organic solvent, to afford the quaternary dirivative $[D\text{-}QC]^+$, which is then reduced by treatment with sodium dithionite or sodium borohydride as generally described hereinabove to afford the desired compound [D-DHC]. When the drug contains more than one reactive hydroxyl or thiol functions, reaction conditions may be varied so that more than one hydroxyl or thiol function will be converted to nicotinate groupings.

The representative drugs listed below may be derivatized in this manner to the corresponding [D-QC]+ and [D-DHC] compounds.

| Starting Material | [D-QC]$^+$ | [D-DHC] |
| --- | --- | --- |
| 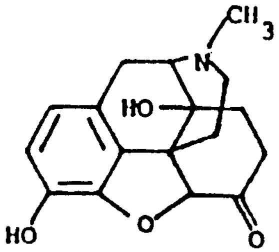<br>oxymorphone | 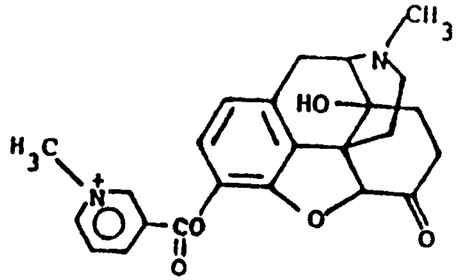 | 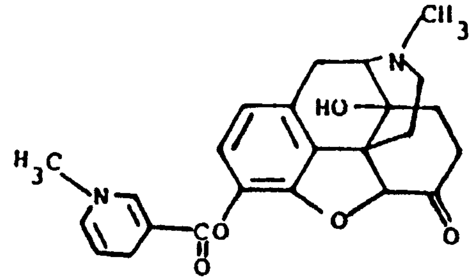 |
| 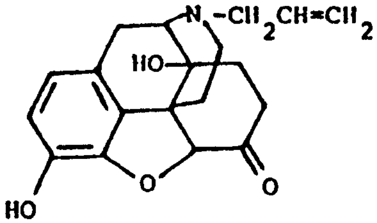<br>naloxone | 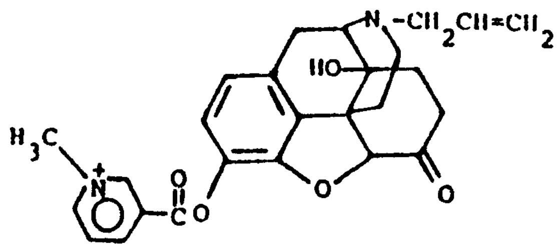 | 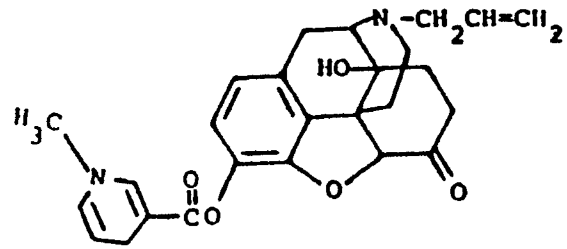 |
| 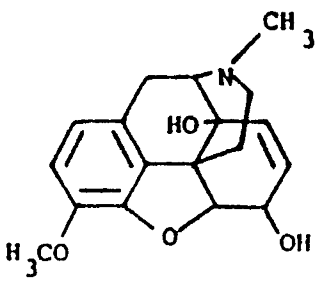<br>oxycodone | 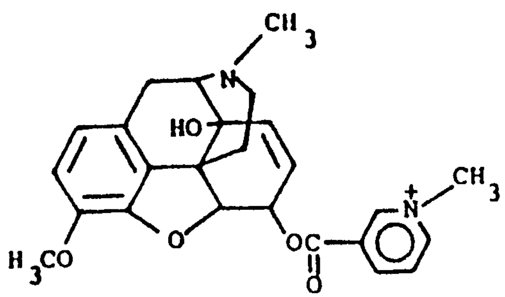 | 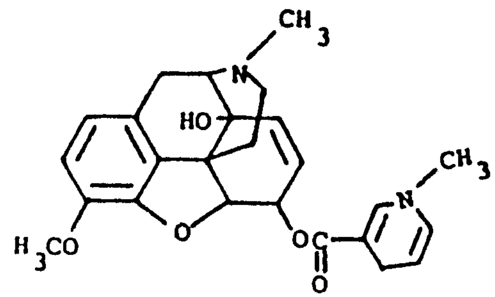 |

| Starting Material | $[D\text{-}QC]^+$ | [D-DHC] |
| --- | --- | --- |
| 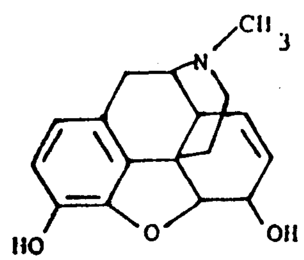 morphine | 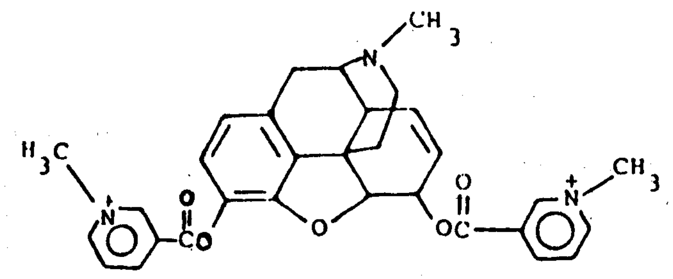 | 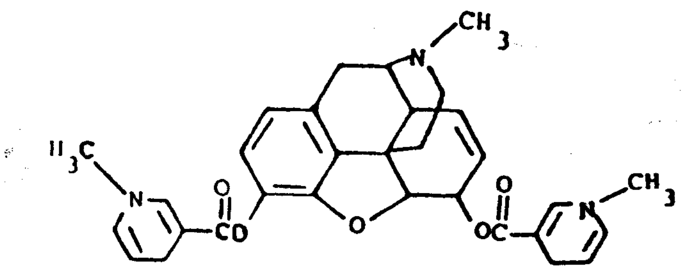 |
| 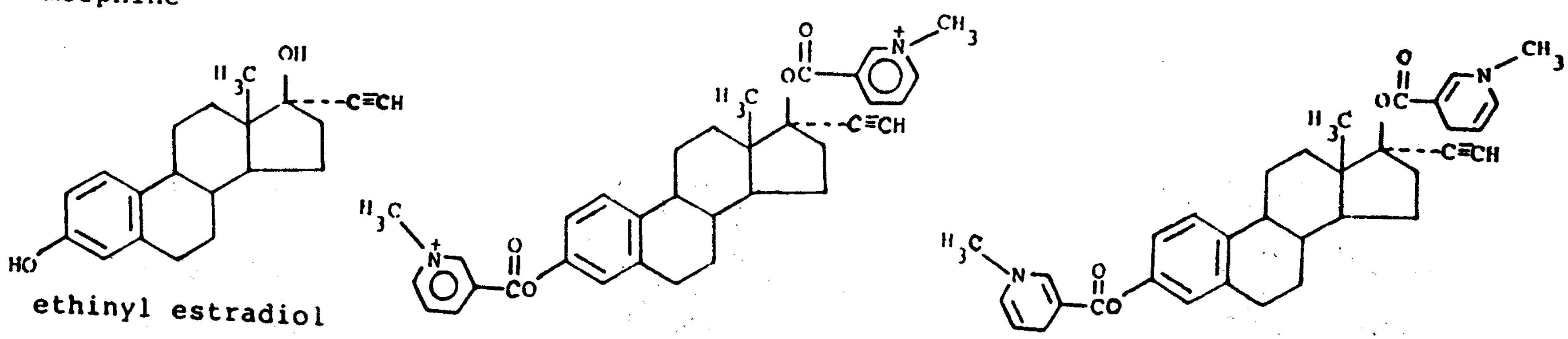 ethinyl estradiol | | |
| 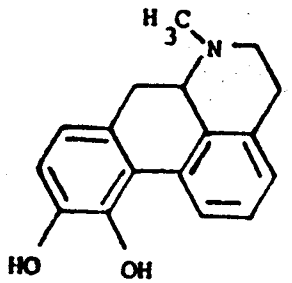 apomorphine | 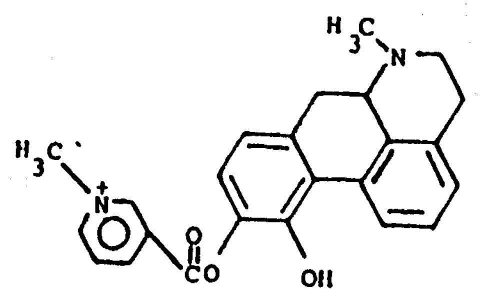 | 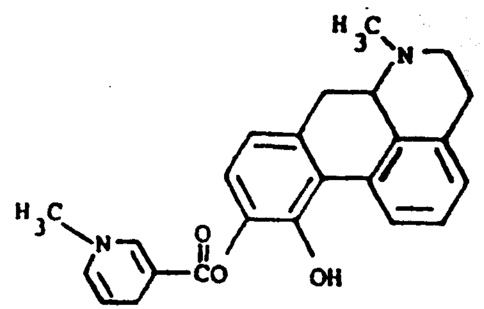 |

| Starting Material | [D-QC]$^{+}$ | [D-DHC] |
| --- | --- | --- |
| 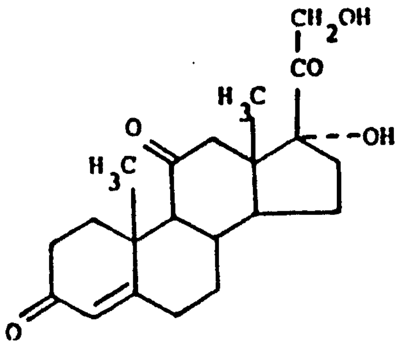<br>cortisone | 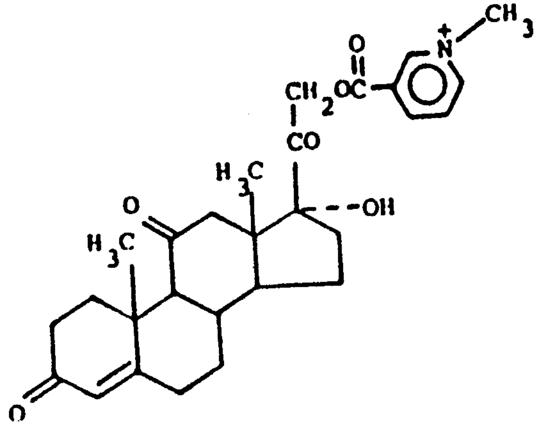 | 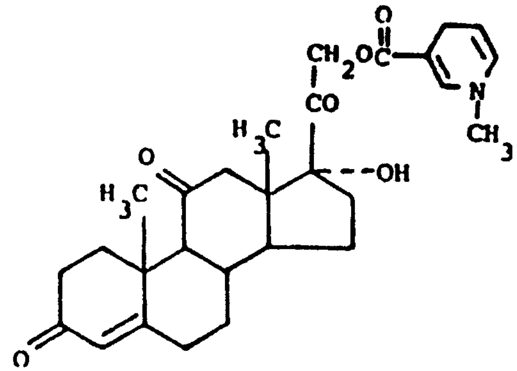 |
| 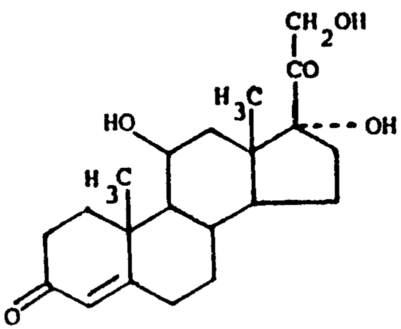<br>hydrocortisone | 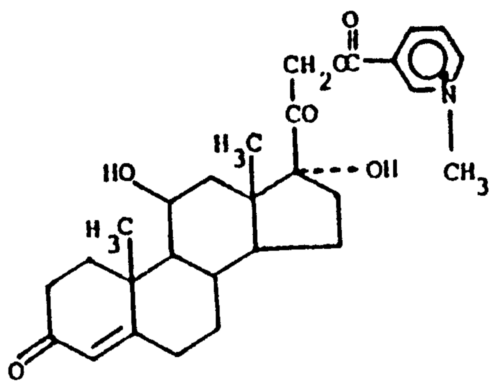 | 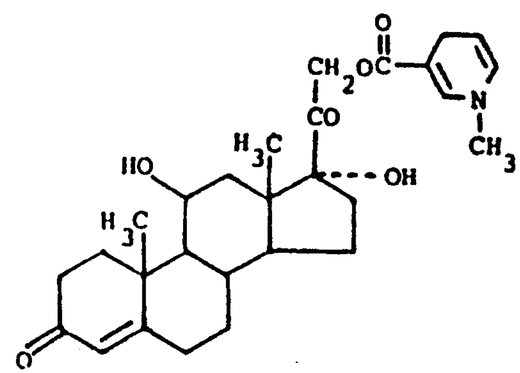 |

| Starting Material | [D-QC]$^+$ | [D-DHC] |
|---|---|---|
| 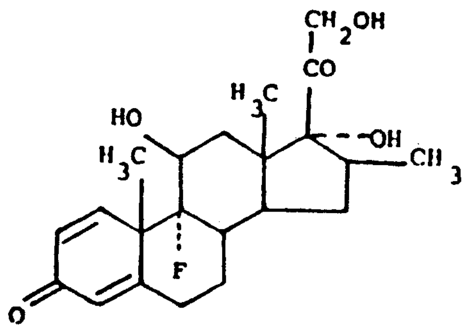 betamethasone | 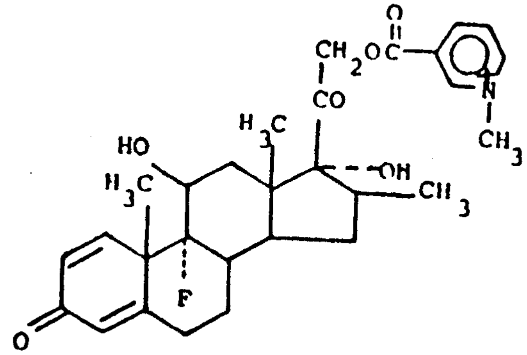 | 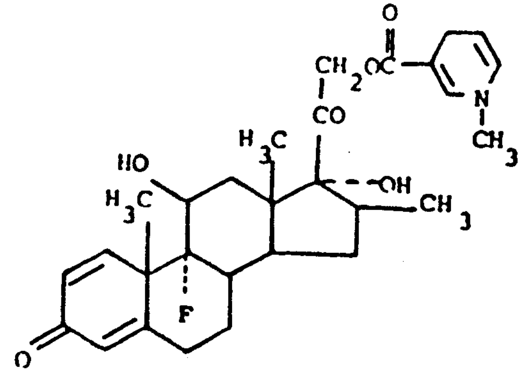 |
| 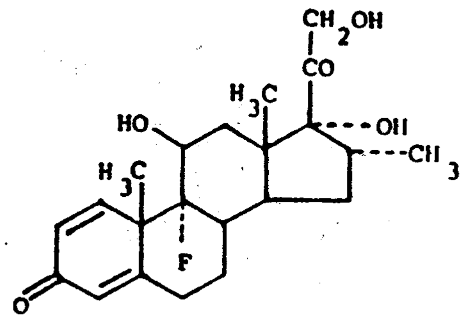 dexamethasone | 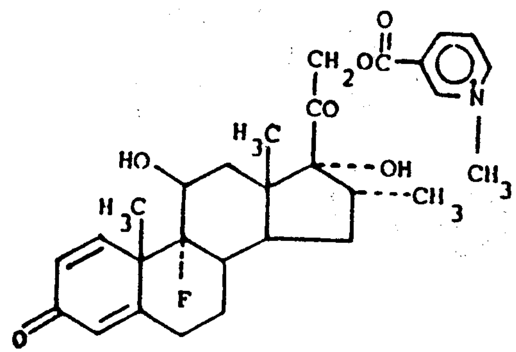 | 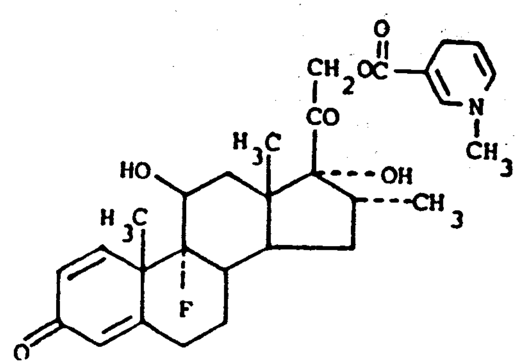 |

| Starting Material | [D-QC]$^+$ | [D-DHC] |
|---|---|---|
| 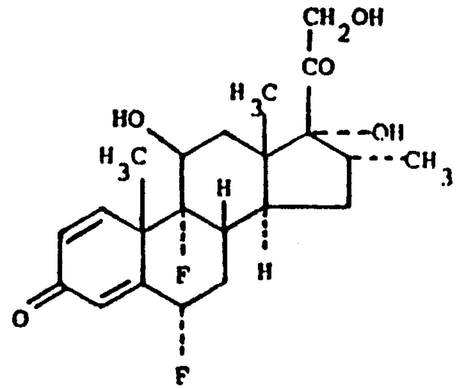<br>flumethasone | 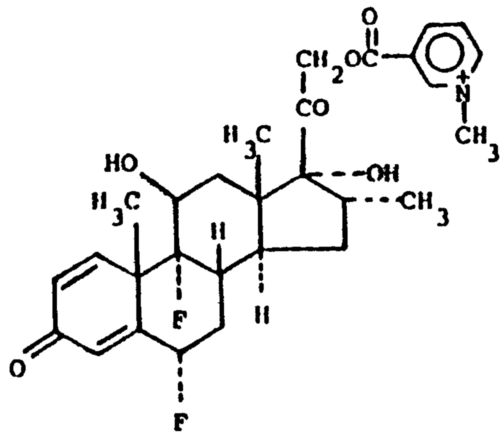 | 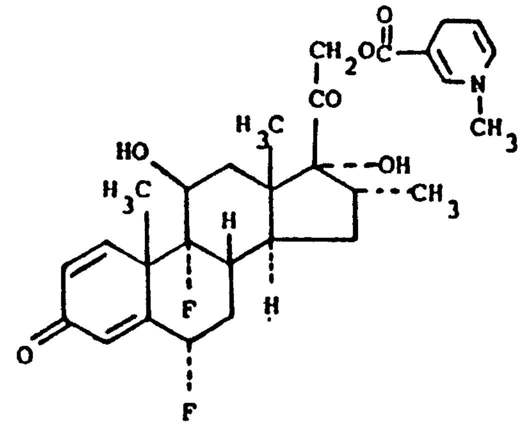 |
| 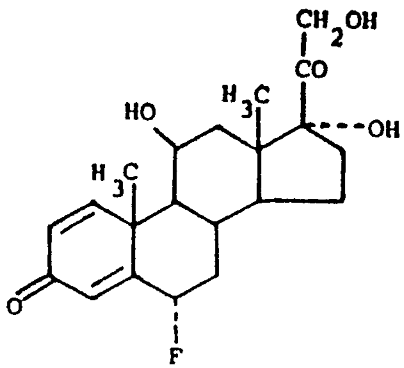<br>fluprednisolone | 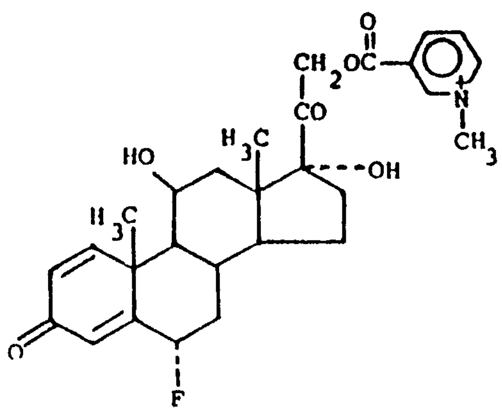 | 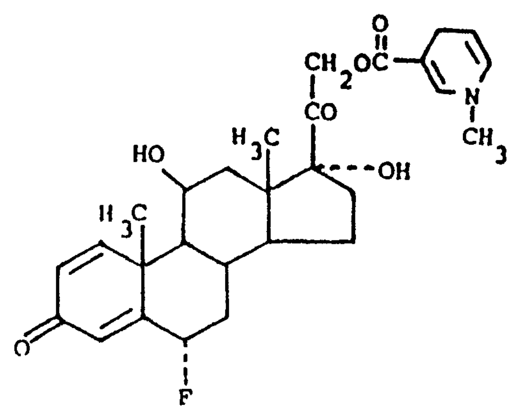 |

| Starting Material | [D-QC]$^+$ | [D-DHC] |
| --- | --- | --- |
| 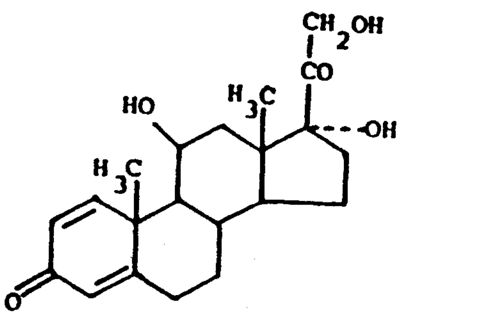 methyl prednisolone | 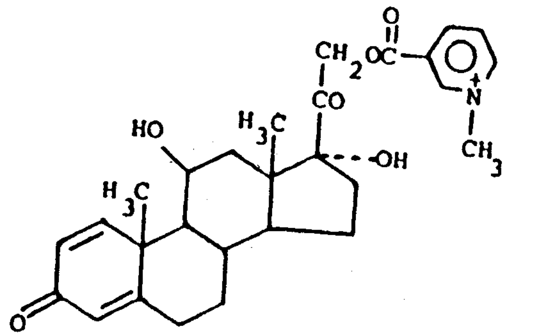 | 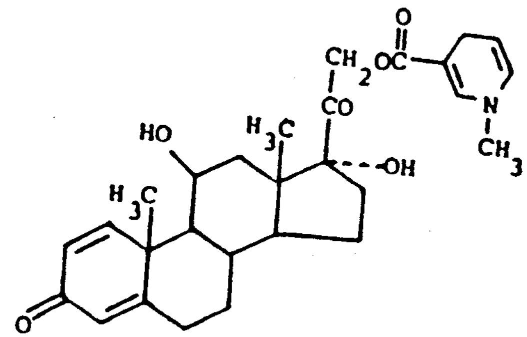 |
| 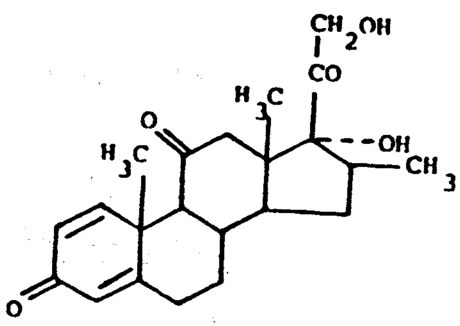 meprednisone | 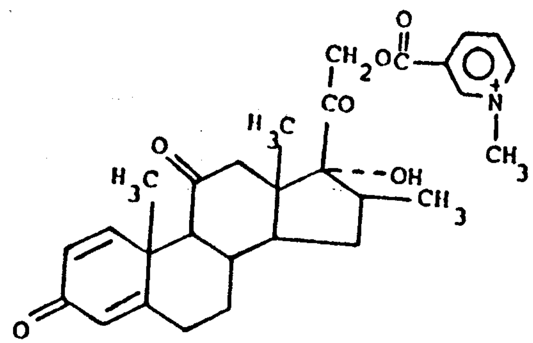 | 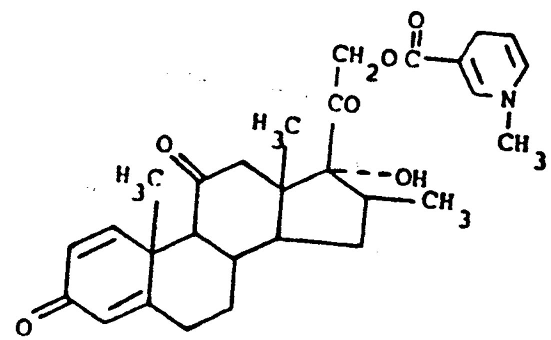 |

| Starting Material | [D-QC]$^+$ | [D-DHC] |
| --- | --- | --- |
| 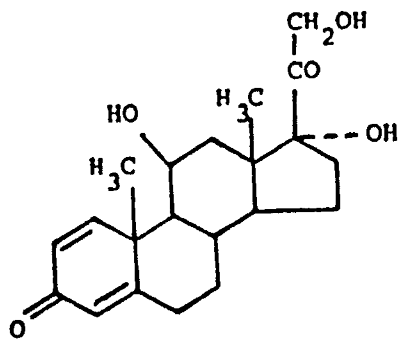 prednisolone | 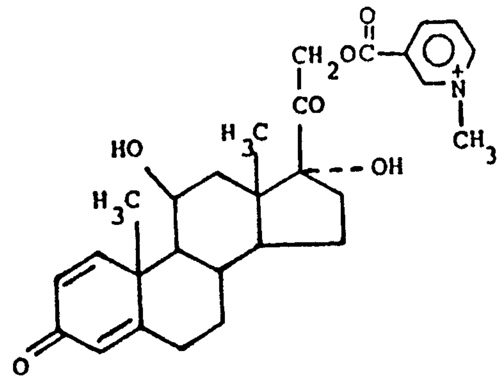 | 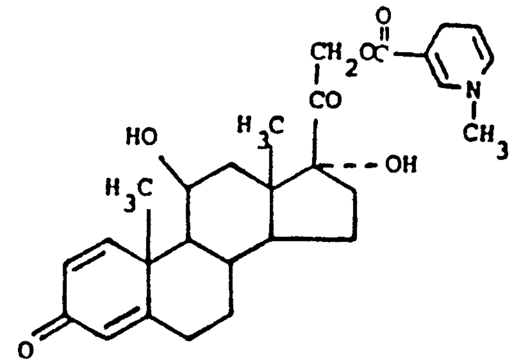 |
| 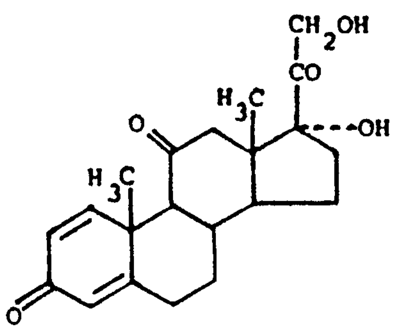 prednisone | 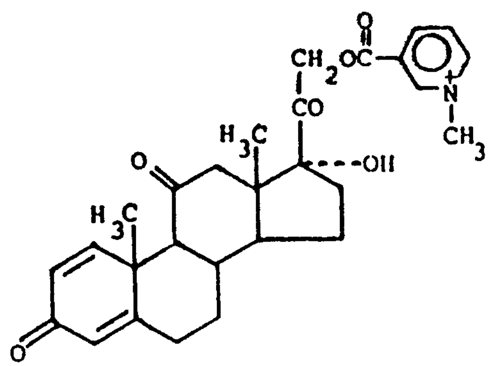 | 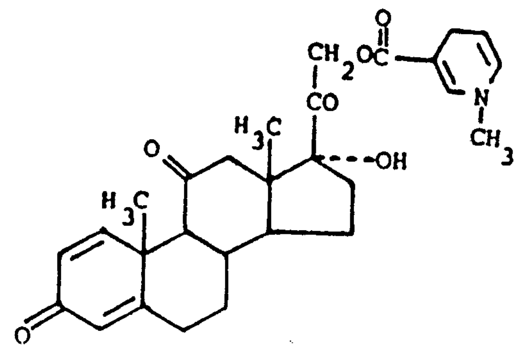 |

| Starting Material | [D-QC]$^+$ | [D-DHC] |
| --- | --- | --- |
| 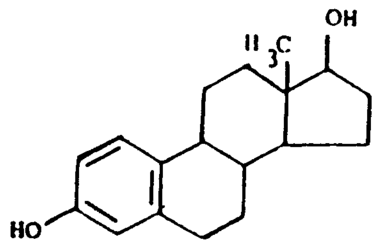 estradiol | 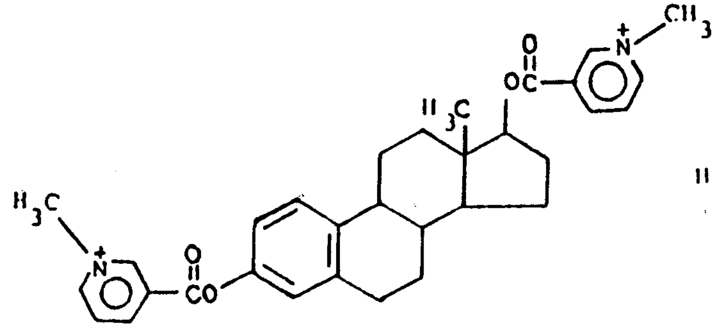 | 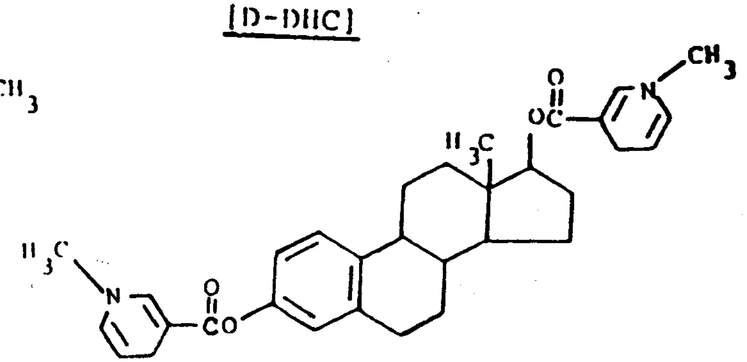 |
| 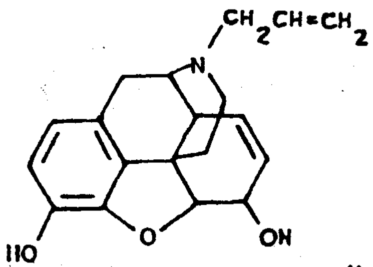 nalorphine | 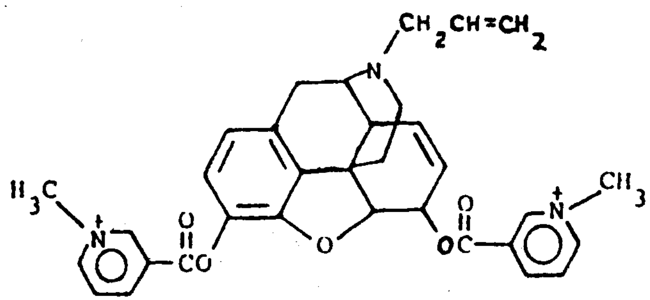 | 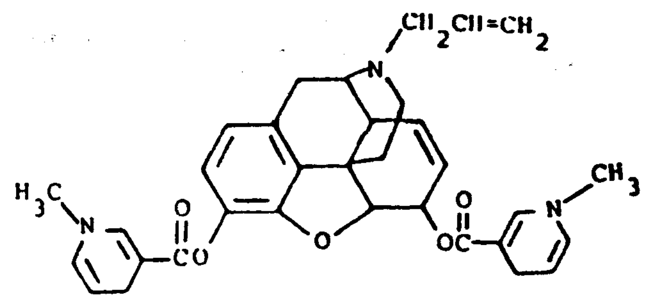 |
| 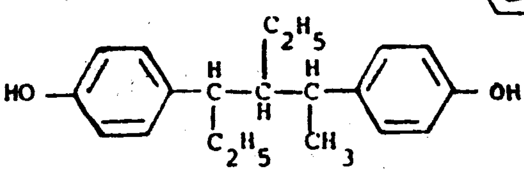 benzestrol | 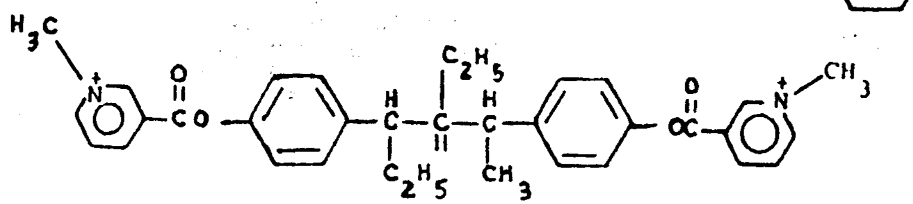 | 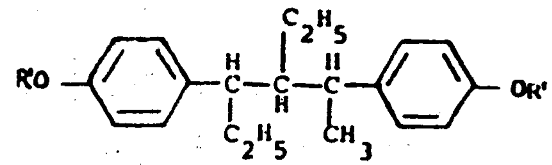 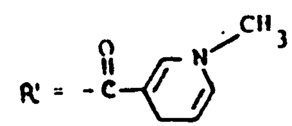 |

Starting Material

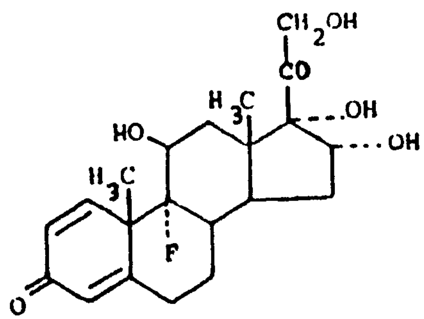

triamcinolone

[D-QC]$^+$

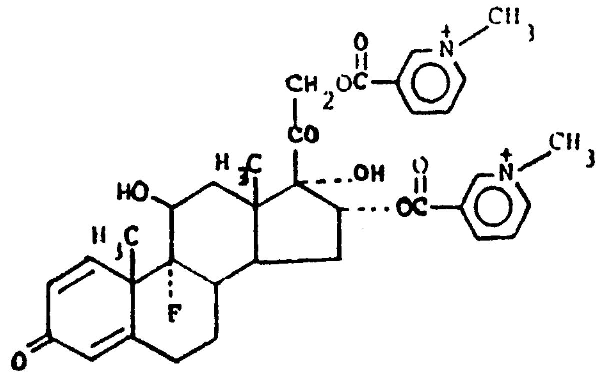

[D-DHC]

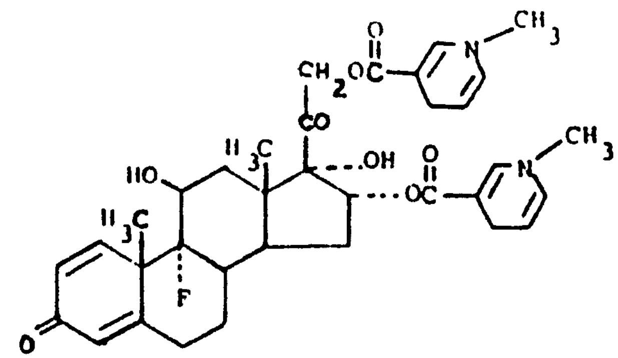

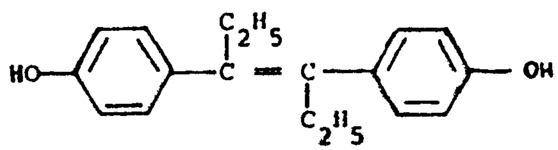

diethylstilbestrol

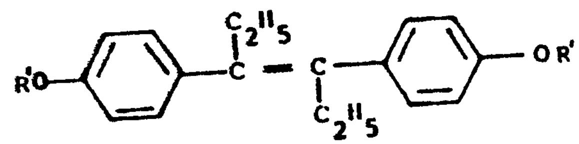

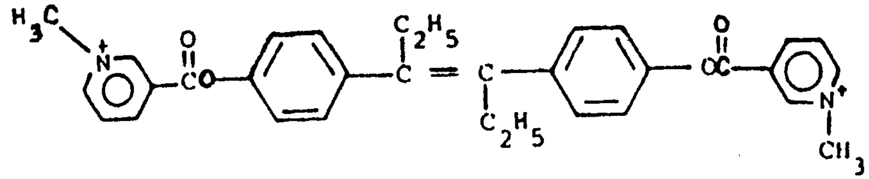

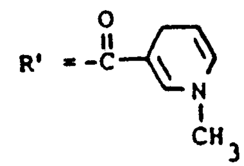

| Starting Material | $[D-QC]^+$ | [D-DHC] |
|---|---|---|
| 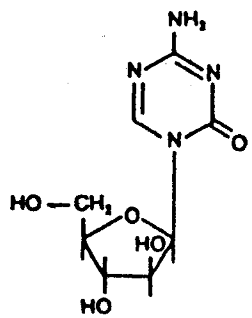<br>Ara-AC | 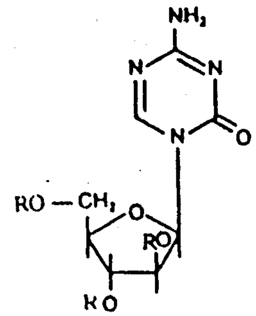 | 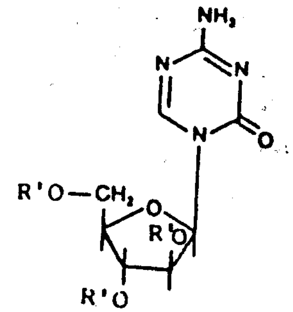 |
| 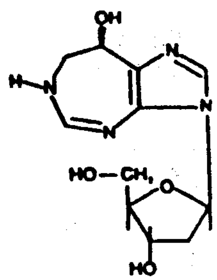<br>pentostatin<br>(2'-deoxycoformycin) | 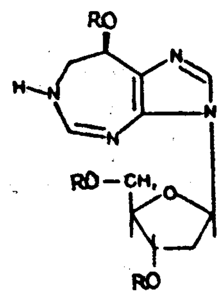 | 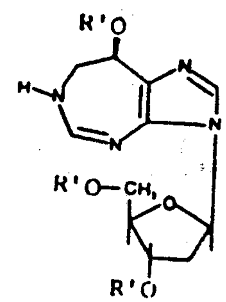 |
| | 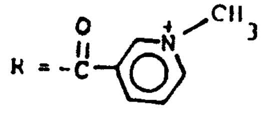 | 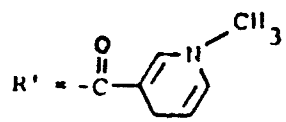 |

| Starting Material | [D-QC]$^+$ | [D-DHC] |
| --- | --- | --- |
| 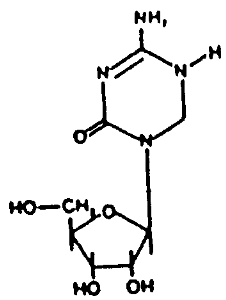<br>dihydro-5-azacytidine | 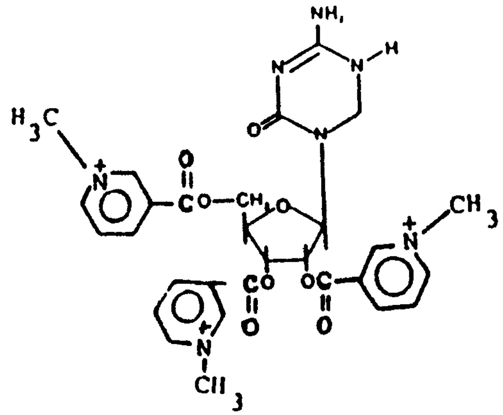 | 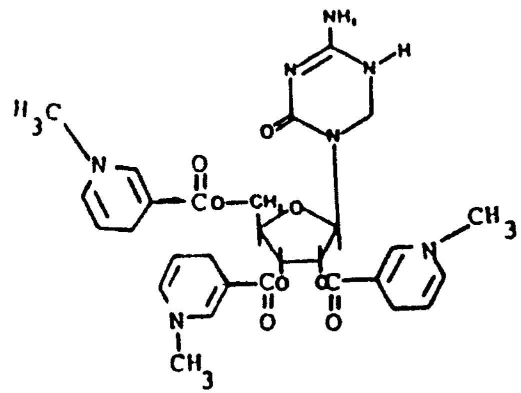 |
| 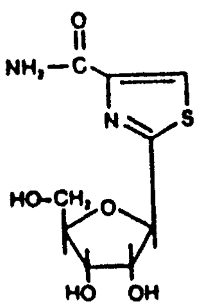<br>tiazofurin | 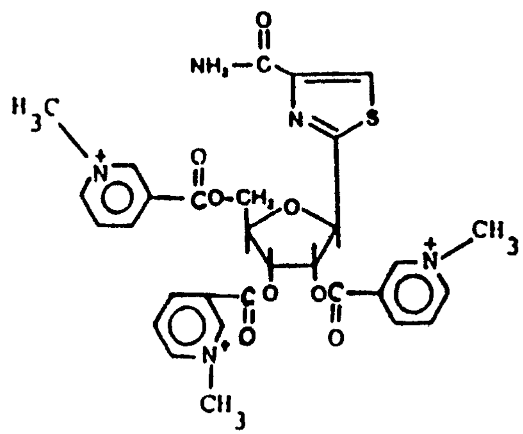 | 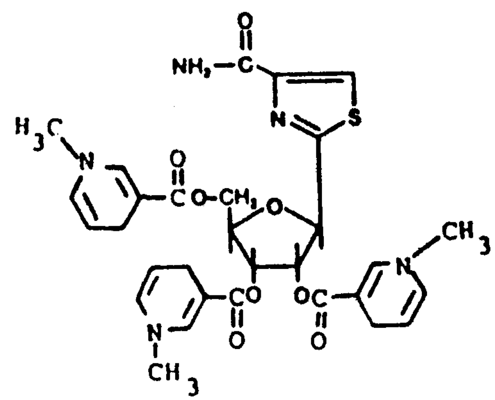 |

## Starting Material

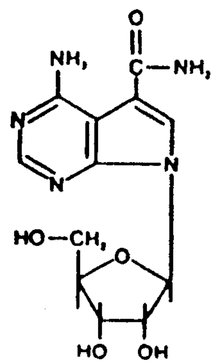

sangivamycin

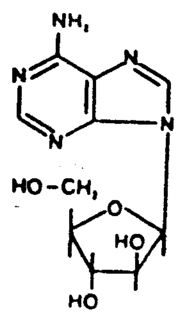

Ara-A

## [D-QC]$^+$

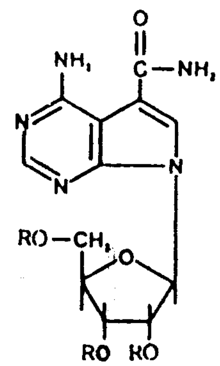

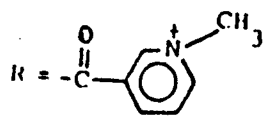

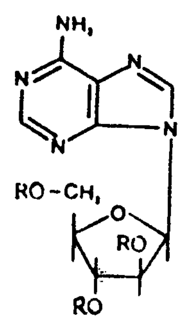

## [D-DHC]

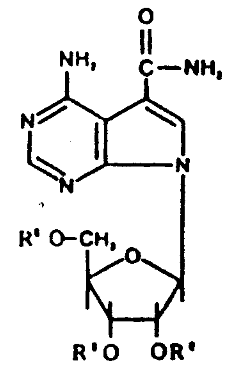

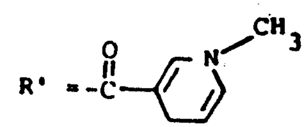

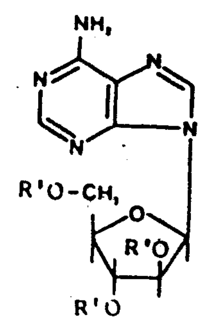

Starting Material | [D-QC]+ | [D-DHC]

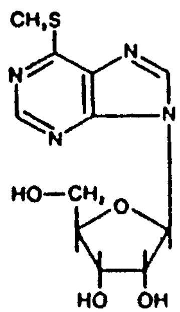

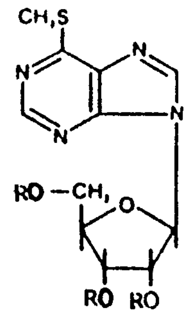

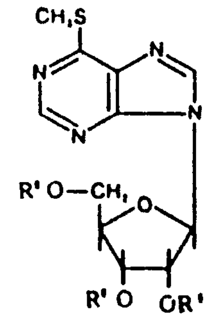

6-MMPR

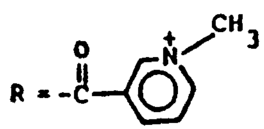

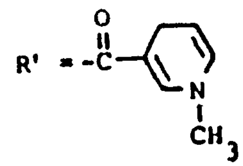

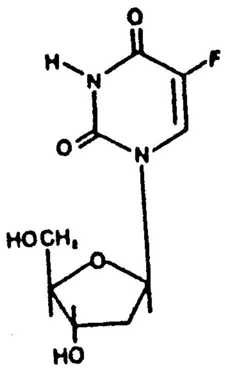

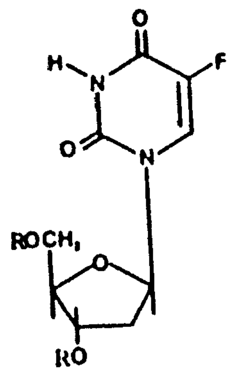

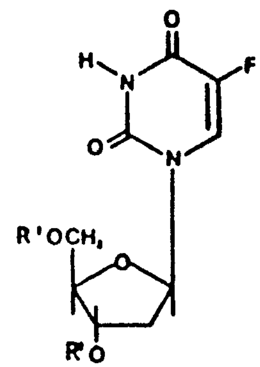

5-FUDR

**Starting Material** | **[D-QC]+** | **[D-DHC]**

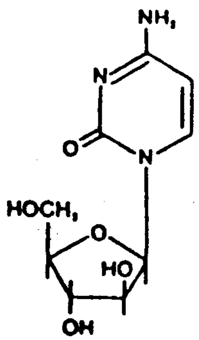

cytosine arabinoside

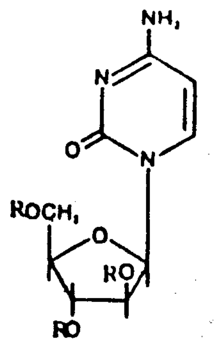

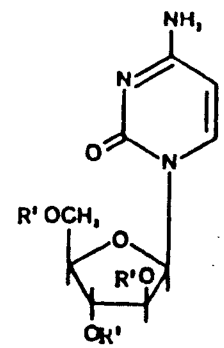

R =
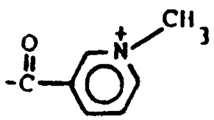

R' =
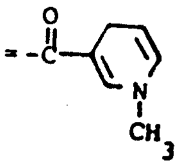

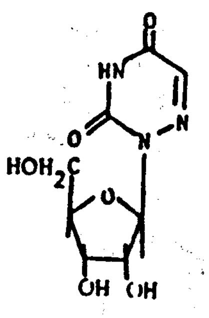

6-azauridine

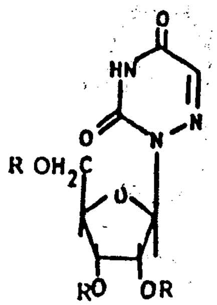

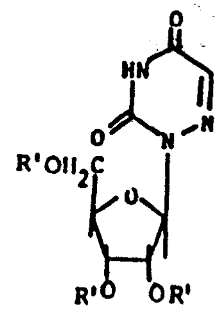

| Starting Material | [D-QC]$^{+}$ | [D-DHC] |
|---|---|---|
| 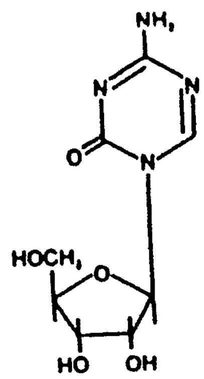 | 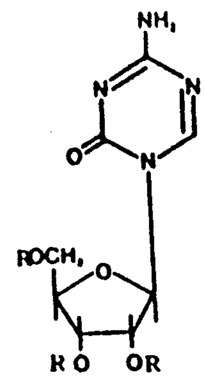 | 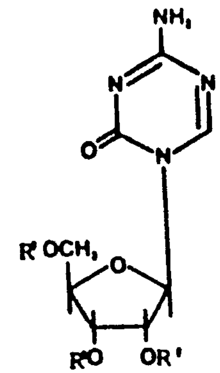 |
| 5-azacytidine | | |
| 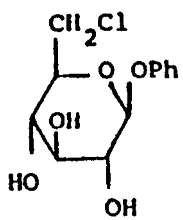 | 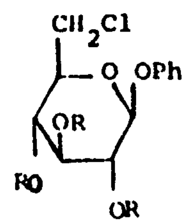 | 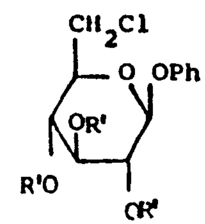 |
| phenyl-6-chloro-6-deoxy-β-D-glucopyranoside | 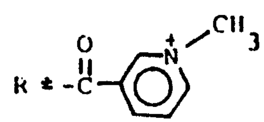 | 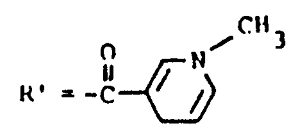 |

| Starting Material | [D-QC]+ | [D-DHC] |
|---|---|---|
| 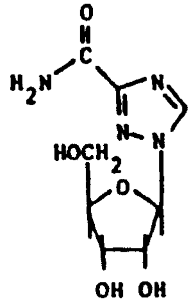 ribavirin | 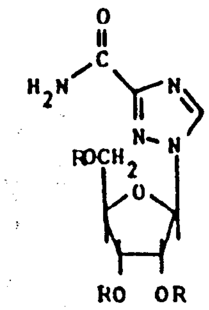 | 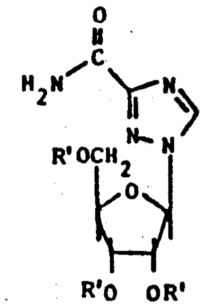 |
| 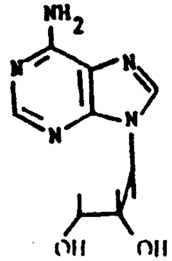 (S)-9-(2,3-dihydroxypropyl)-adenine | 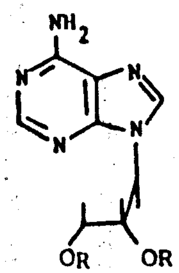 | 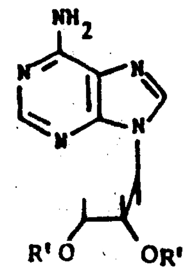 |
| | 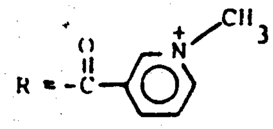 | 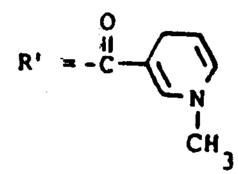 |

## Method B

This is a variation of Method A used when the drug contains a -COOH function which is to be protected.

The drug is first converted to the corresponding ethyl ester by conventional esterification techniques. That ester is then used as the starting material and Method A is repeated. The -COOH group may be similarly converted to other ester groups.

## Method C

Method A is followed, except that in the first step, a starting material of the formula

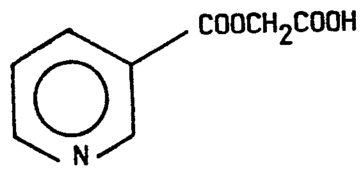

is used in place of nicotinic acid.

The representative drug listed below may be derivatized in this manner to the corresponding $[D\text{-}QC]^+$ and [D-DHC] compounds, as may the remaining drugs listed with Method A.

Similarly, Method C may be combined with Method B to afford the corresponding derivatives.

Starting Material

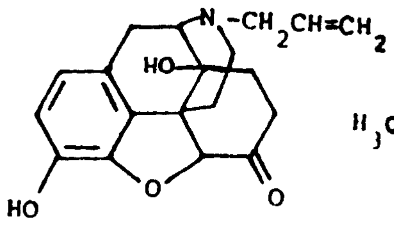

naloxone

[D-QC]$^{+}$

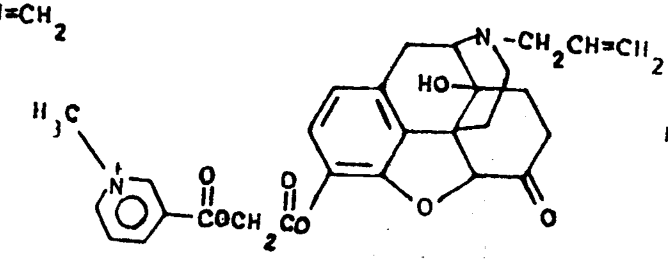

[D-DHC]

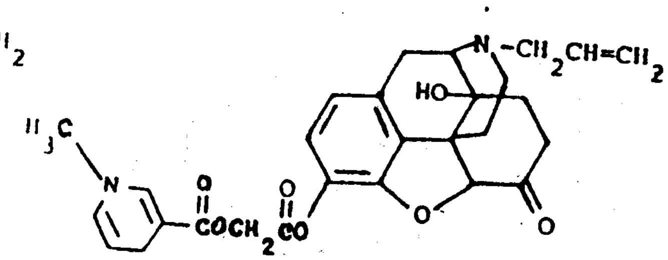

Method for Salt Formation

## Method D

An ether solution of [D-DHC] is treated with an equivalent amount of anhydrous p-toluenesulfonic acid dissolved in dry ether. Mixing at room temperature is continued until the imminium salt precipitates out of solution. The salt is then removed by filtration.

Accordingly, provided hereby are not only a method and novel class of pro-prodrugs for the specific and/or target enhanced delivery to the brain of a wide variety of drug species via the bidirectional transport of the drug species into and out of the brain employing dihydropyridine $\rightleftharpoons$ pyridinium salt carrier redox systems, but also a system providing insight into the basic transport processes (both active and passive) of, and enzymatic activities in, the blood-brain barrier, as well as into the various processes specific to the function of the brain. Again, another very significant aspect of the bioreversible redox delivery system according to this invention is the toxicity implication, for significantly reduced is systemic toxicity by accelerating the elimination of the drug/quaternary carrier system. And even central toxicity is reduced by providing for low level, sustained release of the active drug species in the brain. Low toxicity is provided both as regards the quaternary carrier and in combination with the drug.

CLAIMS:

1. A compound adapted for the site-specific/sustained delivery of a centrally acting drug species to the brain, said compound being a compound of the formula

[D-DHC] (I)

or a non-toxic pharmaceutically acceptable salt thereof, wherein [D] is a centrally acting drug species which is an antiviral agent, an antiinflammatory steroid, a narcotic or an estrogen and which contains at least two reactive hydroxyl functional groups, and [DHC] is the reduced, biooxidizable, blood-brain barrier penetrating lipoidal form of a dihydropyri dine$\rightleftharpoons$pyridinium salt redox carrier comprising at least one dihydropyridine ring system of the formula

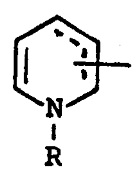

wherein R is lower alkyl or benzyl and the dotted line indicates the presence of a double bond in either the 2 or 3 position of the dihydropyridine ring, a ring carbon atom of each dihydropyridine ring system being connected via a bridging group to a reactive hydroxyl functional group in the centrally acting drug species.

2. A compound according to Claim 1 wherein each dihydropyridine ring system, together with the bridging group connected thereto, comprises the formula

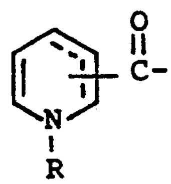

wherein R and the dotted line are as defined in Claim 1.

3. A compound according to Claim 1 or 2 wherein the dotted line indicates the presence of a double bond in the 2 position of the dihydropyridine ring.

4. A compound according to Claim 1 or 2 wherein R is methyl.

5. A compound according to Claim 2 wherein the carboxyl group is attached at the 3 position of the dihydropyridine ring.

6. A compound according to Claim 2 wherein each dihydropyridine ring system, together with the bridging group connected thereto, has the formula

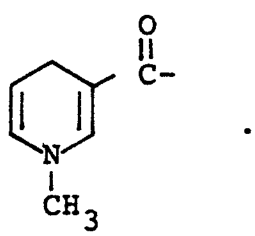

7. A compound according to Claim 2 wherein each dihydropyridine ring system, together with the bridging group connected thereto, has the formula

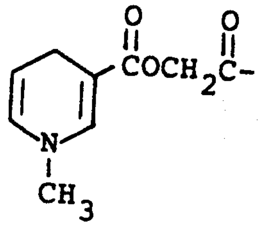

8. A compound of the formula

$$[D\text{-}QC]^+ \qquad (II)$$

wherein [D] is a centrally acting drug species which is an antiviral agent, an antiflammatory steroid, a narcotic or an estrogen and which contains at least two reactive hydroxyl functional groups, and $[QC]^+$ is the hydrophilic, ionic pyridinium salt form of a dihydropyridine $\rightleftharpoons$ pyridinium salt redox carrier comprising at least one pyridinium ring system of the formula

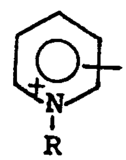

wherein R is lower alkyl or benzyl, a ring carbon atom of each pyridinium ring system being connected via a bridging group to a reactive hydroxyl functional group in the centrally acting drug species.

9. A compound according to Claim 8 wherein each pyridinium ring system, together with the bridging group connected thereto, comprises the formula

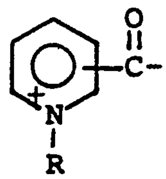

wherein R is as defined in Claim 8.

10. A compound according to Claim 8 or 9 wherein R is methyl.

11. A compound according to Claim 9 wherein the carbonyl group is attached at the 3 position of the pyridinium ring.

12. A compound according to Claim 9 wherein each pyridinium ring system, together with the bridging group connected thereto, has the formula

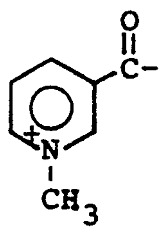

.

13. A compound according to Claim 9 wherein each pyridinium ring system, together with the bridging group connected thereto, has the formula

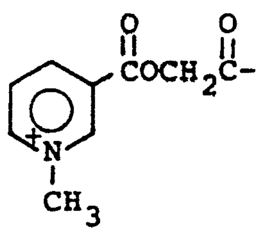

.

14. A compound according to any of the preceding claims wherein [D] is an antiviral agent.

15. A compound according to any of the preceding claims wherein [D] is an antiinflammatory steroid.

16. A compound according to any of the preceding claims wherein [D] is a narcotic analgesic or antagonist.

17. A compound according to any of the preceding claims wherein [D] is an estrogen.

18. A compound according to Claim 1 or 8 wherein [D] is hydrocortisone, betamethasone, dexamethasone, cortisone, flumethasone, fluprednisolone, meprednisone, methyl prednisolone, prednisolone, prednisone, triamcinolone, cortodoxone, fludrocortisone, fluandrenolone acetonide, paramethasone, ethinyl estradiol, estradiol, estriol, Ara-AC, pentostatin, dihydro-5-azacytidine, tiazofurin, sangivamycin, Ara-A, 6-MMPR, 5-FUDR, cytosine arabinoside, 5-azacytidine, ribavirin, 5,7-dimethyl-2-β-D-ribofuranosyl-s-triazole(1,5-a)-pyrimidine, 2-deoxy-D-glucose, glycosamine, 2-deoxy-2-fluoro-D-mannose, 6-amino-6-deoxy-D-glucose, phenyl-6-chloro-6-deoxy-β-D-glucopyranoside, (s)-9-(2,3-dihydroxypropyl)adenine, 6-azauridine, 5,6-dichloro-1-β-D-ribofuranosylbenzimidazole, oxymorphone, apomorphine, morphine, oxycodone, naloxone, nalorphine, benzestrol or diethylstilbestrol.